# FASCICULE DE BREVET EUROPEEN

(11) **EP 2 833 810 B1**
(45) Date de publication et mention de la délivrance du brevet: **07.06.2017**
(21) Numéro de dépôt: 13725389.4
(22) Date de dépôt: 05.04.2013
(51) Int. Cl.: A61B 17/70

(54) **INSTRUMENT ET SYSTEME CHIRURGICAL POUR LA FIXATION DES VERTEBRES**
CHIRURGISCHES INSTRUMENT UND SYSTEM ZUR WIRBELFIXATION
SURGICAL INSTRUMENT AND SYSTEM FOR SECURING VERTEBRAE

(30) Priorité: 05.04.2012 FR 1253132
(43) Date de publication de la demande: 11.02.2015
(73) Titulaire: Safe Orthopaedics, 95610 Eragny-sur-Oise (FR)
(72) Inventeur: PETIT, Dominique, F-62180 Verton (FR)
(74) Mandataire: IP Trust
(86) Numéro de dépôt international: PCT/FR2013/050753
(87) Numéro de publication internationale: WO 2013/150252

(56) Documents cités:
- WO-A2-2011/080426
- US-A1- 2006 111 715
- US-A1- 2012 022 594

## Description

### Domaine de l'invention

La présente invention concerne le domaine des instruments chirurgicaux pour les interventions de stabilisation rachidienne par élément d'ancrage osseux de type vis par voies postérieure ou postéro-latérale.

L'invention concerne plus particulièrement un kit d'instrumentation selon l'invention destiné notamment, mais non exclusivement, à la chirurgie d'ostéosynthèse rachidienne lombaire, thoracique, ou encore cervicale postérieure par voies chirurgicale minimalement invasive ou ouverte.

Lors de dysfonctionnements anatomiques de la colonne vertébrale, on procède à la mise en place d'ancrages osseux de type vis pédiculaire ou vertébrales dans les vertèbres reliées entre elles par des éléments de liaison de type tiges ou plaques.

### Etat de la technique

On connaît dans l'état de la technique la demande de brevet PCT/FR10/000880 (WO2011/080426) de la demanderesse. Ce document divulgue une instrumentation pour la fixation d'au moins deux vertèbres rachidiennes par implants d'ancrage osseux de type vis pédiculaires comprenant un élément d'ancrage osseux destinés à être fixé sur une vertèbre, pré-monté avec sur un tube de montage à usage unique, et un emballage scellé de conditionnement stérile.
Ce document de l'art antérieur concerne également un kit d'instruments pour la pose ou la dépose d'un implant rachidien comportant au moins deux éléments d'ancrage osseux filetés, un organe de liaison de type tige ou plaque reliant mécaniquement les éléments d'ancrage osseux et des éléments de blocage pour verrouiller en position l'élément de liaison par rapport aux éléments d'ancrage, pour réaliser la totalité des gestes chirurgicaux liés à la pose ou la dépose dudit implant, caractérisé en ce que la totalité de ces instruments nécessaires sont à usage unique, conditionnés stérile dans un ou plusieurs emballages scellés.

### Inconvénients de l'état de la technique

Ces solutions de l'art antérieur permettent de simplifier l'acte chirurgical en permettant au chirurgien d'introduire la vis, puis de pousser une tige de liaison intervertébrale, puis de poser un bouchon de verrouillage, avec un seul instrument, qui reste lié à la vis pendant cette succession d'étapes.

Il arrive toutefois que l'instrument de l'art antérieur soit retiré avant la finalisation de l'intervention, par exemple pour permettre une meilleure vision de la zone d'intervention.

La procédure de ré-enclenchement de l'instrument sur la tête de la vis pédiculaire est alors fastidieuse.

Avec l'instrument de l'art antérieur, le ré-enclenchement se fait par un basculement des deux demi-tubes, avec une angulation importante nécessitant une incision de grande taille pour permettre ce mouvement.

Le positionnement de l'extrémité distale se fait avec une faible visibilité par l'opérateur, et la précision du geste est donc très aléatoire.

### Solution apportée par l'invention

La présente invention vise à remédier à cet inconvénient en proposant un instrument chirurgical nouveau, interopérable avec l'instrumentation précitée.

La présente invention propose un instrument chirurgical pour la fixation des vertèbres par voie postérieure ou postéro-latérale selon la revendication indépendante 1. Des réalisations avantageuses de l'invention sont décrites dans les revendications dépendantes. L'invention concerne selon son acception la plus générale un instrument chirurgical pour la fixation des vertèbres par voie postérieure ou postéro-latérale, constitué par :
- un élément tubulaire formé de deux jambages séparés par une zone longitudinale évidée et réunis à leur extrémité distale par un moyen de liaison,
- chacun desdits jambages présentant à sa surface interne une rainure de guidage avec une section en queue d'aronde,
un insert présentant deux glissières longitudinales, diamétralement opposées, présentant une section en queue d'aronde complémentaire à la section desdites rainures de guidage caractérisé en ce que l'extrémité proximale de chacun des jambages présente un moyen d'ancrage apte à venir s'engager dans un moyen complémentaire, par un débattement transversal desdits jambages. Ce débattement se fait selon une direction diamétrale, perpendiculaire à l'axe médian transversal passant entre les deux jambages. En position écartée, les jambages peuvent s'engager sur la tête de la vis par un mouvement axial longitudinal. En position refermée, le moyen d'accrochage empêche le déplacement relatif des jambages et de la tête de la vis.

Il est ainsi possible d'engager l'instrument sur la vis ancrée sur le pédicule par un mouvement d'insertion axial, puis de refermer les jambages, par une action mécanique, par exemple le coulissement d'une bague entourant les jambages, par l'insertion de l'insert qui vient resserrer les deux jambages, ou par retour élastique à la position de repos.

De préférence, l'extrémité proximale de chacun des jambages présente un ergot transversal apte à s'engager dans la gorge présente sur la tête d'une vis pédiculaire, par déformation latérale élastique des jambages.

Selon un mode de réalisation préféré, la hauteur maximale dudit moyen d'ancrage est inférieure à l'ouverture dudit moyen complémentaire.

Avantageusement, ledit ergot présente un épaulement distal s'étendant dans un plan transversal perpendiculaire à l'axe longitudinal de l'insert.

De préférence, ledit ergot présente une surface distale sensiblement conique, pour former un canal d'insertion s'ouvrant en direction proximale, ledit canal présentant en position verrouillée une forme complémentaire à l'enveloppe extérieure de la tête de la vis.

Selon une variante particulière, lesdits jambages divergent, au repos, en direction proximale.

Avantageusement, les jambages sont mobiles entre une position dans laquelle les extrémités proximales sont écartées l'une de l'autre au delà d'une position de repos desdits jambages et une position dans laquelle les extrémités proximales sont resserrées l'une vers l'autre par engagement de l'insert dans la zone longitudinale. Selon une configuration particulière, l'insert resserre les extrémités proximales au-delà de la position au repos des jambages.

Selon une variante préférée, lesdits jambages présentent une forme semi-cylindrique.

Selon un mode de réalisation particulier, ledit moyen de liaison des deux jambages est constitué par une partie distale annulaire prolongeant lesdits jambages.

Selon une autre variante, ledit moyen de liaison des deux jambages est constitué par une poignée reliant lesdits jambages.

L'invention concerne également un système d'instrumentation chirurgical pour la fixation des vertèbres par voie postérieure ou postéro-latérale, constitué par un premier instrument, et un second instrument constitué par un élément tubulaire formé de deux jambages séparés par une zone longitudinale évidée et réunis à leur extrémité distale par un moyen de liaison, chacun desdits jambages présentant à sa surface interne une rainure de guidage avec une section en queue d'aronde, le Système étant caractérisé en outre en ce qu'il comporte ledit insert est commun au premier et au second instrument.

### Description détaillée d'un exemple non limitatif de réalisation

D'autres objets et avantages de l'invention apparaîtront au cours de la description qui suit, faite en référence aux dessins annexés, dans lesquels :
- la figure 1 représente une vue des éléments constitutifs d'un instrument selon l'invention ;
- la figure 2 représente une vue de détail en coupe de l'extrémité des jambages de l'instrument chirurgical selon l'invention
- la figure 3 représente une vue d'une variante de réalisation.

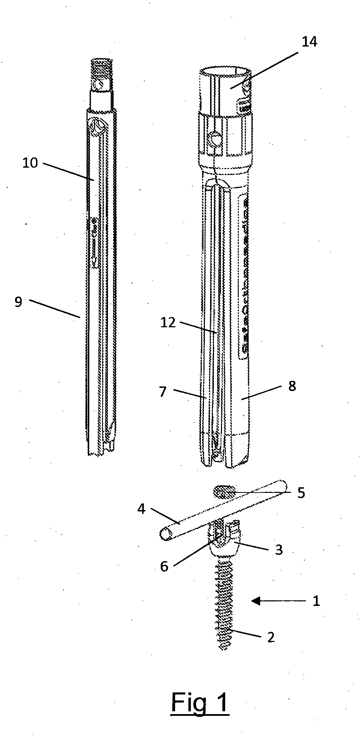

La figure 1 représente une vue d'ensemble des éléments constitutifs d'un instrument chirurgical selon l'invention.

Il comprend une vis pédiculaire (1) destinée à être fixée sur une vertèbre, comprenant un moyen d'ancrage osseux (2) prolongé par une tête (3) fendue pour recevoir une tige de liaison intervertébrale (4). Lorsque la tige (4) est en place, un bouchon (5), coopérant avec la tête (3) par un filetage, vient verrouiller l'ensemble.

Dans une configuration particulière, la tête de la vis (3) est aménagée avec une forme en U (6) destinée à recevoir la tige (4), avec un taraudage intérieur destiné à recevoir le bouchon (4) afin de solidariser la vis (1) et la tige (4). La partie filetée (2) de la vis (1) peut être fixe ou bien mobile vis-à-vis de la tête de la vis (3). Ce type de vis (1) à bouchon (5) appartient au domaine public et constitue un des états de l'art de la chirurgie rachidienne pour la fixation des vertèbres.

Le matériau le plus fréquemment utilisé pour la fabrication des implants est le titane. Dans une configuration particulière de l'invention le matériau utilisé pour la fabrication peut être tous matériaux implantables connus ou non à ce jour, comme le Peek, l'inox, le chrome cobalt, ou encore un composite à base de fibre de verre ou de carbone. Des revêtements de type HATCP (HydroxyApatite TriCalcium Phosphate) ou autres peuvent également être appliqués pour améliorer l'ancrage osseux ou bien la tenue mécanique globale de l'implant.

La pose de cette vis (1) et la mise en place de la tige (4), puis son verrouillage avec le bouchon (5) sont assurés par un instrument décrit dans le brevet de l'art antérieur PCTFR10/000880.

La figure 1 représente un instrument additionnel, objet principal de la présente demande de brevet différent principalement de l'instrument de l'art antérieur par la configuration de l'extrémité proximale (c'est-à-dire proche du coté de la vertèbre et à l'opposé du chirurgien).

Il comprend deux jambages (7, 8) à usage unique, dans l'exemple décrit à titre non limitatif, ainsi qu'un insert (9). L'insert (9) est en tout point identique à celui décrit dans le brevet antérieur PCT/FR10/000880.

Les figures 6 et 7 du brevet antérieur PCT/FR10/000880 montrent le tube porte élément de blocage (8) pré-monté sur le bouchon (3), ainsi que son interconnexion dans le tube de montage (7) de la vis (2).

L'insert (9) est agencé pour maintenir de façon sécurisée le bouchon (5) dans sa partie proximale.

Le maintien du bouchon (5) est assuré par un taraudage prévu à l'extrémité proximale de l'insert (9).

Le maintien du bouchon (5) peut alternativement être assuré par un coincement ou un clipsage.

La forme extérieure de l'insert est pourvue de deux glissières longitudinales (10, 11) présentant une section en queue d'aronde de forme complémentaire à la forme intérieure aux rainures de guidage (12, 13) prévues sur les jambages (7, 8).

Ces queues d'aronde complémentaires entre les deux jambages (7, 8) et l'insert (9) renforcent mécaniquement l'ensemble et évitent ainsi tout risque d'écartement ou de désolidarisation sous contraintes élevées des deux jambages et donnent une résistance de l'ensemble supérieure en flexion et torsion.

L'avantage d'un tel insert est de réaliser simultanément l'étape de pose du bouchon (5) sur la tête (3) de la vis (1) et l'étape de blocage en position des jambages (7, 8) sur la tête de vis. De la même manière, l'insert (9) permet la mise en place de la tige (4) conjointement au blocage en position des deux jambages entre eux.

Les deux jambages (7, 8) présentent une section semi-cylindrique. Leurs parties proximales sont légèrement divergentes, afin de faciliter l'engagement de force sur la tête (3) de la vis par un déplacement longitudinal.

Les deux jambages (7, 8) ne sont pas parallèles mais sont légèrement déviant de façon à éviter le glissement non contraint de l'insert en direction proximale. Le léger angle formé par les deux jambages assure un freinage assurant la stabilisation de l'insert en l'absence d'effort longitudinal exercé par l'utilisateur.

Les jambages (7, 8) sont réunis à leur extrémité distale par un zone annulaire ou par une poignée (14).

Les extrémités proximales sont libres et peuvent donc s'écarter légèrement par déformation élastique. Par contre, lorsque l'insert (9) est engagé et avancé en direction proximale, il vient resserrer les deux jambages et assurer la liaison mécanique. Ainsi, une fois en place dans la zone longitudinale, l'insert (9) fige la position des jambages (7, 8) l'un par rapport à l'autre.

Il en est de même avec la bague (18) de la variante de réalisation décrite plus loin.

L'action de l'insert et de la bague est identique. Le chirurgien a la possibilité d'assurer le verrouillage en agissant sur la bague et/ou sur l'insert. Le chirurgien peut par exemple assurer un premier verrouillage en déplaçant la bague en position proximale, puis en confortant le verrouillage en engageant l'insert.

Les jambages (7, 8) et l'insert (9) peuvent être fabriqués dans tout matériaux connus à ce jour comme les composites, les polymères, les métaux ferreux, et non ferreux (aluminium) tant que ceux-ci correspondent aux critères de biocompatibilité liés à l'application. De préférence, le matériau utilisé sera recyclable afin de répondre aux exigences de protection de l'environnement. Des revêtements peuvent également être appliqués afin de répondre aux critères de biocompatibilité ou bien accroitre les caractéristiques mécaniques.

Le mode de stérilisation choisi sera compatible avec les caractéristiques desdits matériaux suivant la technique de l'art. Cette stérilisation se fera de préférence suivant une irradiation Gamma ou encore suivant un procédé spécifique à l'oxyde d'éthylène (ETO).

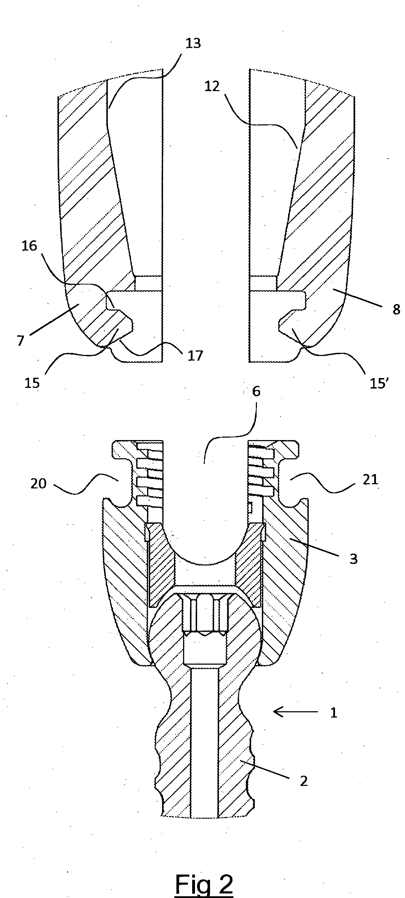

La figure 2 représente une vue détaillée de l'extrémité proximale des jambages (7, 8).

Elle présente un ergot (15, 15') s'étendant dans une direction transversale. La hauteur de cet ergot est définie pour permettre son engagement latéral dans la gorge (20, 21)) prévue sur la tête de la vis.

L'ergot présente une face distale (16) plane, légèrement inclinée avec une pente d'environ 40 degrés par rapport au plan transversal. Cette face distale (16) forme un épaulement apte à coopérer avec un épaulement complémentaire proximal de la tête de la vis. Cette coopération permet d'assurer un ancrage longitudinal de l'instrument sur la tête de la vis. Il est ainsi possible d'exercer des efforts axiaux sans désolidariser l'instrument de la vis (1), par exemple pour pousser la tige (4) dans la fente en U de la vis (1).

L'ergot (15) présente également une face proximale (17) formant un chanfrein, avec une pente d'environ 30 degrés par rapport au plan transversal, pour faciliter l'engagement par clipsage sur la tête de la vis.

### Variante de réalisation

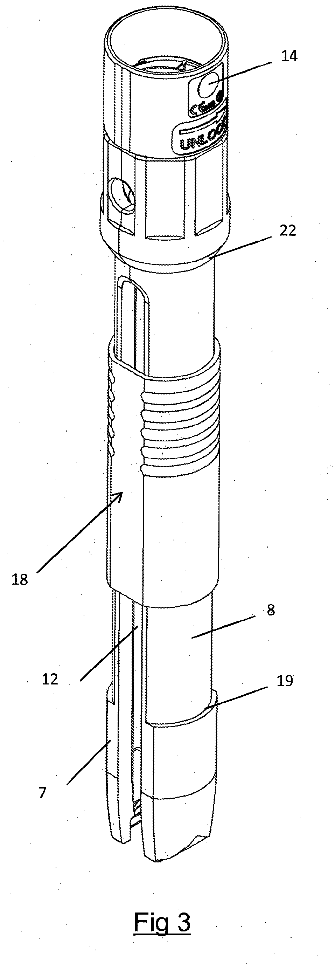

La figure 3 représente une variante de réalisation d'un instrument, où les jambages (7, 8) sont entourés par une bague (18) coulissante. La section intérieure de la bague est complémentaire de l'enveloppe extérieure des jambages (7, 8). Elle présente deux arcs semi-circulaires reliés par des tronçons droits ronde si les jambages sont demi-cylindriques séparés par une ouverture pour le passage de la tige intervertébrale.

La hauteur de la bague (18) est par exemple de 50 millimètres de long.

L'un des jambages (ou les deux) présente des butées proximales (19) et distales (22) de limitation de course de la bague (18). La butée proximale est située à environ 30 millimètres de l'extrémité proximale. La butée distale est constituée par l'élément de liaison (14).

Lorsque la bague (18) est en position distale, les jambages peuvent être écartés pour venir s'engager sur la tête de la vis. Lorsque la bague (18) est repoussée en position proximale, elle ressert les jambages (7, 8) sur la tête de la vis, et assure le verrouillage de l'instrument sur la tête de la vis.

### Système comprenant un instrument principal et un instruments additionnel selon l'invention

L'instrument chirurgical selon l'invention peut faire partie d'un kit d'instruments à usage unique, conditionné stérile pour la pose ou la dépose des implants.

L'insert est alors commun à l'instrument de base et à l'instrument additionnel objet du présent brevet.

Le kit peut comporter plusieurs autres instruments et le cas échéant des implants permettant de réaliser la totalité des gestes chirurgicaux exigés pour la pose ou la dépose des implants.

Ce dispositif kit d'instruments, lorsqu'il est réalisé en matériaux polymères stériles à usage unique, comporte beaucoup d'avantages comme celui de réduire le coût global de la chirurgie du rachis, et de garantir la non contamination d'un patient à un autre, et donc de réduire significativement le nombre d'infection nosocomiale.

De façon non limitative ni restrictive, le kit d'instruments permet d'effectuer les gestes chirurgicaux suivants: la mise en place des vis dans les pédicules des vertèbres, le cintrage de la tige pour être en conformité avec l'anatomie du patient, la mise en place de la tige quelques soient les efforts d'introduction, la mise en place du bouchon, les manoeuvres de correction des vertèbres instrumentées de type compression et distraction, le serrage final contrôlé et sécurisé du bouchon.

## Revendications

1. Instrument chirurgical pour la fixation des vertèbres par voie postérieure ou postéro-latérale, constitué par :
• un élément tubulaire formé de deux jambages (7, 8) séparés par une zone longitudinale évidée, lesdits jambages (7, 8) étant reliés à leur extrémité distale,
• chacun desdits jambages (7, 8) présentant à sa surface interne une rainure de guidage (12, 13) avec une section en queue d'aronde,
• un insert (9) présentant deux glissières longitudinales (10, 11), diamétralement opposées, présentant une section en queue d'aronde complémentaire à la section desdites rainures de guidage,
l'extrémité proximale de chacun des jambages (7, 8) présentant un moyen d'ancrage (15) arrangé pour venir s'engager dans un moyen complémentaire (20, 21) prévu sur la tête d'une vis pédiculaire par un débattement transversal desdits jambages, **caractérisé en ce que**, au repos, lesdits jambages (7, 8) divergent en direction proximale pour éviter le glissement non contraint de l'insert en direction proximale.

2. Instrument chirurgical pour la fixation des vertèbres par voie postérieure ou postéro-latérale selon la revendication 1, **caractérisé en ce que** l'extrémité proximale de chacun des jambages (7, 8) présente un ergot (15, 15') transversal apte à s'engager dans la gorge (20, 21) présente sur la tête de la vis pédiculaire, par déformation latérale élastique des jambages (7, 8).

3. Instrument chirurgical selon la revendication 2, **caractérisé en ce que** ledit ergot (15) présente une surface proximale (17) sensiblement conique, pour former un canal d'insertion s'ouvrant en direction distale, ledit canal présentant en position verrouillée une forme complémentaire à l'enveloppe extérieure de la tête de la vis.

4. Instrument chirurgical selon la revendication 2 ou la revendication 3, **caractérisé en ce que** l'ergot (15) présente une face proximale (17) formant un chanfrein présentant une pente d'environ 30 degrés par rapport au plan transversal.

5. Instrument chirurgical selon l'une quelconque des revendications précédentes, **caractérisé en ce que** les jambages (7, 8) sont mobiles entre une position dans laquelle les extrémités proximales sont écartées l'une de l'autre au delà d'une position de repos desdits jambages (7, 8) et une position dans laquelle les extrémités proximales sont resserrées l'une vers l'autre par engagement de l'insert (9) dans la zone longitudinale.

6. Instrument chirurgical selon l'une quelconque des revendications précédentes, **caractérisé en ce que** lesdits jambages (7, 8) présentent une forme semi-cylindrique.

7. Instrument chirurgical selon la revendication 1 à 6, **caractérisé en ce que** moyen de liaison des deux jambages (7, 8) constitué par une partie distale annulaire prolongeant lesdits jambages (7, 8).

8. Instrument chirurgical selon la revendication 1 à 6, **caractérisé en ce que** moyen de liaison des deux jambages (7, 8) constitué par une poignée reliant lesdits jambages (7, 8).

9. Instrument chirurgical selon l'une quelconque des revendications précédentes, **caractérisé en ce que** les deux jambages (7, 8) forment des pièces séparées, reliées à leur extrémité distale par un moyen de liaison (14).

10. Instrument chirurgical selon l'une quelconque des revendications précédentes, **caractérisé en ce qu'**il comporte en outre une bague (18) entourant les deux jambages (7, 8), la dite bague (18) étant mobile longitudinalement entre une position proximale où elle assure le verrouillage des jambages (7, 8) sur la tête de la vis, et une position distale où elle permet le déplacement transversale des jambages (7, 8) en vue la désolidarisation de la tête de la vis.

11. Système d'instrumentation chirurgical pour la fixation des vertèbres par voie postérieure ou postéro-latérale, constitué par un premier instrument conforme à la revendication 1, et un second instrument constitué par un élément tubulaire formé de deux jambages séparés par une zone longitudinale évidée et réunis à leur extrémité distale par un moyen de liaison, chacun desdits jambages présentant à sa surface interne une rainure de guidage avec une section en queue d'aronde, le système étant **caractérisé en outre en ce qu'**il comporte ledit insert est commun au premier et au second instrument.

## Patentansprüche

1. Chirurgisches Instrument für die Befestigung von Wirbeln auf hinterem oder hinterem-seitlichem Weg, gebildet aus:
* einem röhrenförmigen Element, das aus zwei Stützen (7, 8) gebildet ist, die durch einen ausgesparten Längsbereich getrennt sind, wobei die genannten Stützen (7, 8) an ihren distalen Enden verbunden sind,
* wobei jede der genannten Stützen (7, 8) an ihrer internen Fläche eine Führungsrille (12, 13) mit einem schwalbenschwanzförmigen Querschnitt aufweist,
* einem Einsatz (9), der zwei genau entgegengesetzte längliche Gleitschienen (10, 11) aufweist, die einen schwalbenschwanzförmigen Querschnitt aufweisen, der zum Querschnitt der genannten Führungsrillen komplementär ist,
wobei das proximale Ende jeder Stütze (7, 8) ein Verankerungsmittel (15) aufweist, das angeordnet ist, um in ein komplementäres Mittel (20, 21) einzugreifen, das auf dem Kopf einer pedikulären Schraube durch ein transversales Ausfedern der genannten Stützen vorgesehen ist, **dadurch gekennzeichnet, dass** die genannten Stützen (7, 8) in Ruhestellung in der proximalen Richtung abweichen, um das Gleiten ohne Beanspruchung des Einsatzes in proximaler Richtung zu verhindern.

2. Chirurgisches Instrument für die Befestigung von Wirbeln auf hinterem oder hinterem-seitlichem Weg gemäß Anspruch 1, **dadurch gekennzeichnet, dass** das proximale Ende jeder Stütze (7, 8) einen transversalen Nocken (15, 15') aufweist, der geeignet ist, per lateraler elastischer Verformung der Stützen (7, 8) in die Auskehlung (20, 21) einzugreifen, die auf dem Kopf der pedikulären Schraube vorhanden ist.

3. Chirurgisches Instrument gemäß Anspruch 2, **dadurch gekennzeichnet, dass** der genannte Nocken (15) eine proximale Fläche (17) aufweist, die deutlich konisch ist, um einen Einführungskanal zu bilden, der sich in distaler Richtung öffnet, wobei der genannte Kanal in verriegelter Position eine zur äußeren Hülle des Kopfs der Schraube komplementäre Form aufweist.

4. Chirurgisches Instrument gemäß Anspruch 2 oder Anspruch 3, **dadurch gekennzeichnet, dass** der Nocken (15) eine proximale Seite (17) aufweist, die eine Fase bildet, die eine Neigung von ungefähr 30 Grad im Verhältnis zur transversalen Ebene aufweist.

5. Chirurgisches Instrument gemäß irgendeinem der voranstehenden Ansprüche, **dadurch gekennzeichnet, dass** die Stützen (7, 8) zwischen einer Position, in der die proximalen Enden voneinander über eine Ruheposition der genannten Stützen (7, 8) hinweg beabstandet sind, und einer Position, in der die proximalen Enden zueinander per Eingreifen des Einsatzes (9) in den länglichen Bereich verengt sind.

6. Chirurgisches Instrument gemäß irgendeinem der voranstehenden Ansprüche, **dadurch gekennzeichnet, dass** die genannten Stützen (7, 8) eine halbzylindrische Form haben.

7. Chirurgisches Instrument gemäß Anspruch 1 bis 6, **dadurch gekennzeichnet, dass** das distale Ende der zwei Stützen (7, 8) ein Verbindungsmittel der zwei Stützen (7, 8) aufweist, das durch einen ringförmigen distalen Teil gebildet ist, der die genannten Stützen (7, 8) verlängert.

8. Chirurgisches Instrument gemäß Anspruch 1 bis 6, **dadurch gekennzeichnet, dass** das distale Ende der zwei Stützen (7, 8) ein Verbindungsmittel der zwei Stützen (7, 8) aufweist, das durch einen Griff gebildet ist, der die genannten Stützen (7, 8) verbindet.

9. Chirurgisches Instrument gemäß irgendeinem der voranstehenden Ansprüche, **dadurch gekennzeichnet, dass** die zwei Stützen (7, 8) separate Teile bilden, die an ihrem distalen Ende durch ein Verbindungsmittel (14) verbunden sind.

10. Chirurgisches Instrument gemäß irgendeinem der voranstehenden Ansprüche, **dadurch gekennzeichnet, dass** es darüber hinaus einen Ring (18) umfasst, der die zwei Stützen (7, 8) umgibt, wobei der genannte Ring (18) in Längsrichtung zwischen einer proximalen Position, in der er die Verriegelung der Stützen (7, 8) auf dem Kopf der Schraube sichert, und einer distalen Position, in der er die transversale Verschiebung der Stützen (7, 8) zur Loslösung des Kopfes von der Schraube zulässt, mobil ist.

11. Chirurgisches Instrumentensystem für die Befestigung von Wirbeln auf hinterem oder hinterem-seitlichem Weg, das durch ein erstes Instrument gemäß Anspruch 1 gebildet ist, und ein zweites Instrument, das durch ein röhrenförmiges Element gebildet ist, das durch zwei Stützen geformt ist, die durch einen ausgesparten länglichen Bereich getrennt und an ihrem distalen Ende durch ein Verbindungsmittel vereint sind, wobei jede der genannten Stützen an ihrer internen Fläche eine Führungsrille mit einem schwalbenschwanzförmigen Querschnitt aufweist, wobei das System darüber hinaus **dadurch gekennzeichnet ist, dass** es den genannten Einsatz umfasst, der dem ersten und dem zweiten Instrument gemeinsam ist.

## Claims

1. A surgical instrument for fastening vertebrae via the posterior approach or via the posterolateral approach, the instrument being constituted by:
• a tubular element formed of two legs (7, 8) separated by a recessed longitudinal zone, said legs (7, 8) being interconnected at their distal ends;
• each of said legs (7,8) having, in its inside surface, a guide channel (12, 13) of dovetail cross-section; and
• an insert (9) having two diametrically opposite longitudinal slideways (10, 11) of dovetail cross-section complementary to the cross-section of said guide channels;
the proximal end of each of the legs (7, 8) having anchor means (15) arranged to come to engage in complementary means (20, 21) provided in the head of a pedicle screw by a transverse movement of said legs, said surgical instrument being **characterized in that**, at rest, said legs (7, 8) diverge in the proximal direction in such a manner as to avoid unstressed slippage of the insert in the proximal direction.

2. A surgical instrument for fastening vertebrae via the posterior approach or via the posterolateral approach according to claim 1, **characterized in that** the proximal end of each of the legs (7, 8) is provided with a transverse lug (15, 15') suitable for being engaged in a groove (20, 21) in the head of the pedicle screw, by lateral elastic deformation of the legs (7, 8).

3. A surgical instrument according to claim 2, **characterized in that** said lug (15) has a proximal surface (17) that is substantially conical, for forming an insertion canal that opens out in the distal direction, said canal having, in the locked position, a shape complementary to the outside envelope of the head of the screw.

4. A surgical instrument according to claim 2 or claim 3, **characterized in that** the lug (15) has a proximal face (17) forming a bevel, with a slope of about 30 degrees relative to the transverse plane.

5. A surgical instrument according to any preceding claim, **characterized in that** the legs (7, 8) are mounted to move between a position in which the proximal ends are spaced apart from each other beyond a rest position of said legs (7, 8), and a position in which the proximal ends are brought closer together by the insert (9) being engaged in the longitudinal zone.

6. A surgical instrument according to any preceding claim, **characterized in that**, said legs (7, 8) are of semicylindrical shape.

7. A surgical instrument according to any one of claims 1 to 6, **characterized in that** the distal ends of the two legs (7, 8) have interconnection means for interconnecting the two legs (7, 8), which interconnection means are constituted by an annular distal portion extending said legs (7, 8).

8. A surgical instrument according to any one of claims 1 to 6, **characterized in that** the distal ends of the two legs (7, 8) have interconnection means for interconnecting the two legs (7, 8), which interconnection means are constituted by a handle that interconnects said legs (7, 8).

9. A surgical instrument according to any preceding claim, **characterized in that** the two legs (7, 8) form separate parts, interconnected at their distal ends by interconnection means (14).

10. A surgical instrument according to any preceding claim, **characterized in that** it further comprises a ring (18) surrounding the two legs (7, 8), said ring (18) being mounted to move longitudinally between a proximal position in which it locks the legs (7, 8) on the head of the screw and a distal position in which it enables the legs (7, 8) to move transversely with a view to releasing the head of the screw.

11. A surgical instrument system for fastening vertebrae via the posterior approach or via the posterolateral approach, said system being constituted by a first instrument according to claim 1, and by a second instrument constituted by a tubular element formed of two legs separated by a recessed longitudinal zone and joined at their distal ends by interconnection means, each of said legs having, in its inside surface, a guide groove of dovetail cross-section, the system further being **characterized in that** said insert is common to the first instrument and to the second instrument.
